# EUROPEAN PATENT APPLICATION

(11) **EP 4 331 488 A1**
(43) Date of publication of application: **06.03.2024**
(21) Application number: 23188408.1
(22) Date of filing: 28.07.2023
(51) Int. Cl.: A61B 5/316, A61B 5/349, A61B 5/352, A61B 5/00, G16H 40/67, G16H 50/20

(54) **METHOD AND SYSTEM FOR GENERATING 2D REPRESENTATION OF ELECTROCARDIOGRAM (ECG) SIGNALS**

(30) Priority: 05.09.2022 IN 202221050616
(71) Applicant: Tata Consultancy Services Limited, Maharashtra (IN)
(72) Inventor: UKIL, ARIJIT, 700160 Kolkata - West Bengal (IN); GUBBI LAKSHMINARASIMHA, JAYAVARDHANA RAMA, 560066 Bangalore - Karnataka (IN); PAL, ARPAN, 700160 Kolkata - West Bengal (IN); DEB, TRISROTA, 700160 Kolkata - West Bengal (IN); RACHA, SAI CHANDER, 500081 Hyderabad - Telangana (IN); SAHU, ISHAN, 700160 Kolkata - West Bengal (IN); KHANDELWAL, SUNDEEP, 201309 Noida - Uttar Pradesh (IN)
(74) Representative: Goddar, Heinz J.

(57) **Abstract**

Portable ECG monitors available in market have the disadvantage that the ECG data they provide as input aren't directly interpretable and requires medical knowledge for the users. The disclosure herein generally relates to Electrocardiogram (ECG), and, more particularly, to a method and system for generating 2d representation of electrocardiogram (ECG) signals. The system provides a mechanism for determining variability between a plurality of segments of an ECG data measured, and uses the information on the determined variability to generate the 2D representation corresponding to the ECG signal. The system further provides means to generate a data model that can be further used for processing real-time ECG data for generating corresponding interpretations. This allows a user to obtain the interpretations as output.

## Description

### CROSS-REFERENCE TO RELATED APPLICATIONS AND PRIORITY

The present application claims priority from Indian application no. 202221050616, filed on September 5, 2022.

### TECHNICAL FIELD

The disclosure herein generally relates to Electrocardiogram (ECG), and, more particularly, to a method and system for generating 2D representation of electrocardiogram (ECG) signals.

### BACKGROUND

Electrocardiogram (ECG) signal is a recording of heart's electrical activity, and plays an important role in representing health of a person. In healthcare clinics or hospitals, ECG measurement is taken using sophisticated ECG monitors, and trained professionals interpret the ECG signals and detect variations that may require medical assistance.

However, these days, mainly from convenience point of view, users prefer having smaller versions of essential health monitoring devices at own residences. Many portable ECG monitors are available in market and they provide ECG measurements to the users. However, unlike blood sugar measurements or blood pressure measurements which are directly interpretable, ECG signals aren't directly interpretable and require medical knowledge. This affects the larger uptake of the technology.

### SUMMARY

Embodiments of the present disclosure present technological improvements as solutions to one or more of the above-mentioned technical problems recognized by the inventors in conventional systems. For example, in one embodiment, a processor implemented method is provided. In this method, a raw Electrocardiogram (ECG) signal is obtained as input via one or more hardware processors. Further, noise data from the raw ECG signal is removed via a bandpass filter implemented by the one or more hardware processors, to obtain a clean signal. Further, the clean signal is segmented via the one or more hardware processors, to obtain a plurality of segments, wherein each of the plurality of segments comprises a left R-peak and a right R-peak. Further, the plurality of segments are arranged by aligning the left R-peak of the plurality of segments. Further, variability between the plurality of segments is determined in terms of position of the left R-peak and the right R-peak of consecutive segments, via the one or more hardware processors. Further, 2-Dimensional (2D) representation of the segments is generated, via the one or more hardware processors, wherein in the 2D representation, information on relative position of the left R-peak and the right R-peak of each of the segments are captured.

In another aspect, a system is provided. The system includes one or more hardware processors, a communication interface, and a memory comprising a plurality of instructions. The plurality of instructions cause the one or more hardware processors to obtain a raw Electrocardiogram (ECG) signal as input. Further, noise data from the raw ECG signal is removed via a bandpass filter implemented by the one or more hardware processors, to obtain a clean signal. Further, the clean signal is segmented via the one or more hardware processors, to obtain a plurality of segments, wherein each of the plurality of segments comprises a left R-peak and a right R-peak. Further, the plurality of segments are arranged by aligning the left R-peak of the plurality of segments. Further, variability between the plurality of segments is determined in terms of position of the left R-peak and the right R-peak of consecutive segments, via the one or more hardware processors. Further, 2-Dimensional (2D) representation of the segments is generated, via the one or more hardware processors, wherein in the 2D representation, information on relative position of the left R-peak and the right R-peak of each of the segments are captured.

In yet another aspect, a non-transitory computer readable medium is provided. The non-transitory computer readable medium includes a plurality of instructions, which when executed, cause one or more hardware processors to obtain a raw Electrocardiogram (ECG) signal as input. Further, noise data from the raw ECG signal is removed via a bandpass filter implemented by the one or more hardware processors, to obtain a clean signal. Further, the clean signal is segmented via the one or more hardware processors, to obtain a plurality of segments, wherein each of the plurality of segments comprises a left R-peak and a right R-peak. Further, the plurality of segments are arranged by aligning the left R-peak of the plurality of segments. Further, variability between the plurality of segments is determined in terms of position of the left R-peak and the right R-peak of consecutive segments, via the one or more hardware processors. Further, 2-Dimensional (2D) representation of the segments is generated, via the one or more hardware processors, wherein in the 2D representation, information on relative position of the left R-peak and the right R-peak of each of the segments are captured.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 illustrates an exemplary system for generating 2D representation of ECG signals, according to some embodiments of the present disclosure.
FIG. 2 is a flow diagram depicting steps involved in the process of generating 2D representation of ECG signals, using the system of FIG. 1, according to some embodiments of the present disclosure.
FIG. 3 is a flow diagram depicting steps involved in the process of generating a data model for generating 2D representation of ECG signals, using the system of FIG. 1, according to some embodiments of the present disclosure.
FIGS. 4A through 4G are example diagrams depicting the process of generating 2D representation of ECG signals, according to some embodiments of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Many portable ECG monitors are available in market and they provide ECG measurements to the users. However, unlike blood sugar measurements or blood pressure measurements which are directly interpretable, ECG signals aren't directly interpretable and require medical knowledge. This affects larger uptake of the technology.

To address this problem, embodiments disclosed herein provide a method and system for generating 2D representation of electrocardiogram (ECG) signals that enables easy interpretability to humans and allows end to end automation of ECG analysis. The system provides a mechanism for determining variability between a plurality of segments of an ECG data measured, and uses the information on the determined variability to generate the 2D representation corresponding to the ECG signal. The system further provides means to generate a data model that can be further used for processing real-time ECG data for generating corresponding interpretations. This allows a user to obtain the interpretations as output.

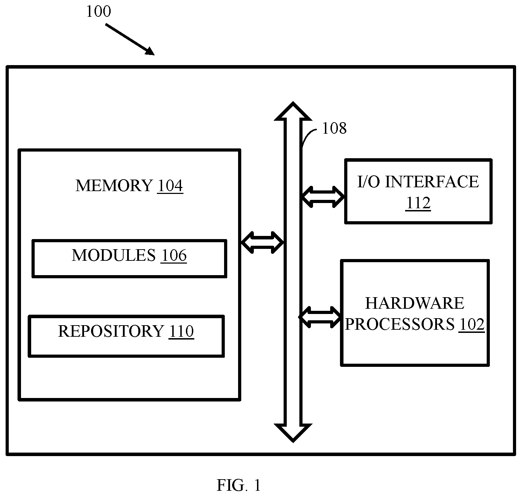

Referring now to the drawings, and more particularly to FIG. 1 through FIG. 4B, where similar reference characters denote corresponding features consistently throughout the figures, there are shown preferred embodiments and these embodiments are described in the context of the following exemplary system and/or method.

FIG. 1 illustrates an exemplary system for generating 2D representation of ECG signals, according to some embodiments of the present disclosure. The system 100 includes or is otherwise in communication with hardware processors 102, at least one memory such as a memory 104, an I/O interface 112. The hardware processors 102, memory 104, and the Input /Output (I/O) interface 112 may be coupled by a system bus such as a system bus 108 or a similar mechanism. In an embodiment, the hardware processors 102 can be one or more hardware processors.

The I/O interface 112 may include a variety of software and hardware interfaces, for example, a web interface, a graphical user interface, and the like. The I/O interface 112 may include a variety of software and hardware interfaces, for example, interfaces for peripheral device(s), such as a keyboard, a mouse, an external memory, a printer and the like. Further, the I/O interface 112 may enable the system 100 to communicate with other devices, such as web servers, and external databases.

The I/O interface 112 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface 112 may include one or more ports for connecting several computing systems with one another or to another server computer. The I/O interface 112 may include one or more ports for connecting several devices to one another or to another server.

The one or more hardware processors 102 may be implemented as one or more microprocessors, microcomputers, microcontrollers, digital signal processors, central processing units, node machines, logic circuitries, and/or any devices that manipulate signals based on operational instructions. Among other capabilities, the one or more hardware processors 102 is configured to fetch and execute computer-readable instructions stored in the memory 104.

The memory 104 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes. In an embodiment, the memory 104 includes a plurality of modules 106.

The plurality of modules 106 include programs or coded instructions that supplement applications or functions performed by the system 100 for executing different steps involved in the process of generating the 2D representation of ECG signals, being performed by the system 100. The plurality of modules 106, amongst other things, can include routines, programs, objects, components, and data structures, which performs particular tasks or implement particular abstract data types. The plurality of modules 106 may also be used as, signal processor(s), node machine(s), logic circuitries, and/or any other device or component that manipulates signals based on operational instructions. Further, the plurality of modules 106 can be used by hardware, by computer-readable instructions executed by the one or more hardware processors 102, or by a combination thereof. The plurality of modules 106 can include various sub-modules (not shown). The plurality of modules 106 may include computer-readable instructions that supplement applications or functions performed by the system 100 for generating the 2D representation of ECG signals.

The data repository (or repository) 110 may include a plurality of abstracted piece of code for refinement and data that is processed, received, or generated as a result of the execution of the plurality of modules in the module(s) 106.

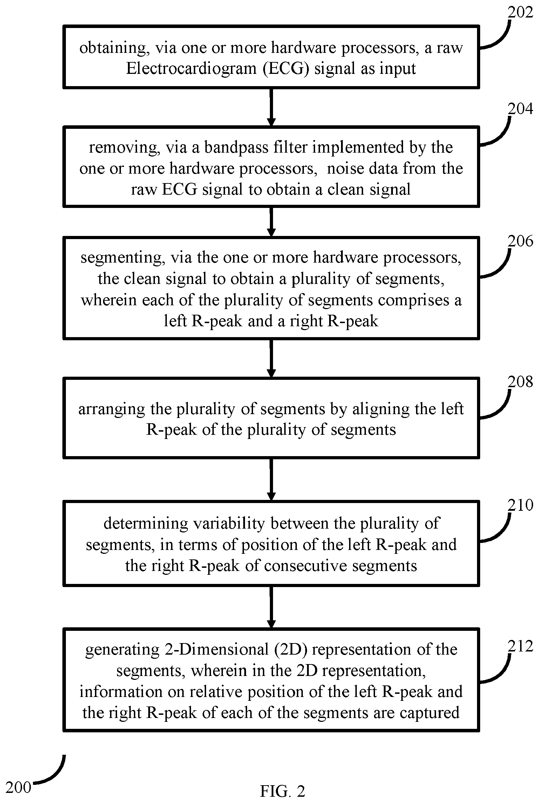

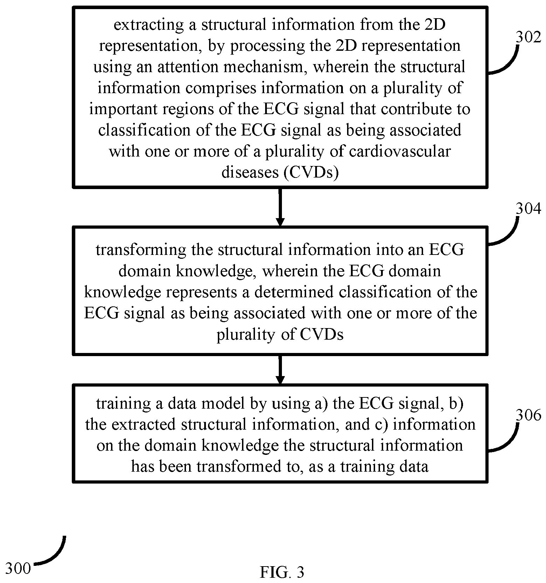

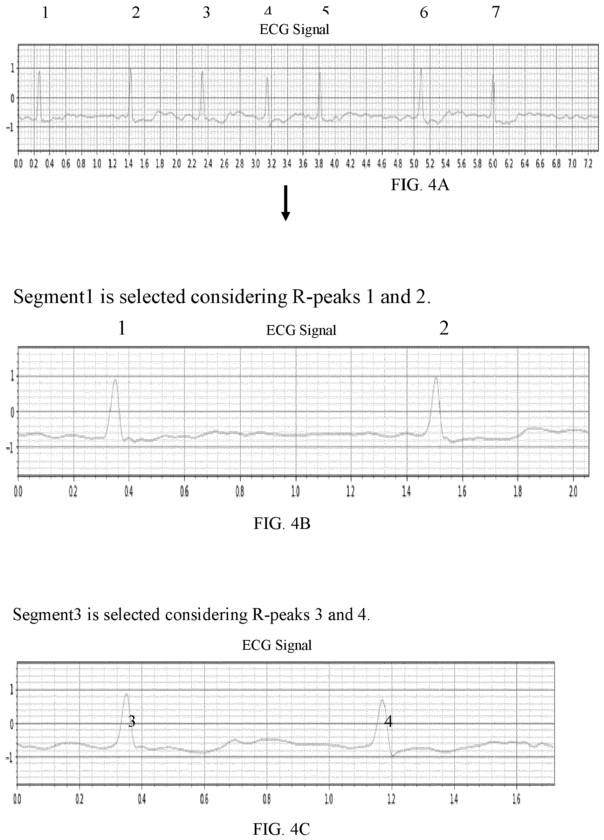

Although the data repository 110 is shown internal to the system 100, it will be noted that, in alternate embodiments, the data repository 110 can also be implemented external to the system 100, where the data repository 110 may be stored within a database (repository 110) communicatively coupled to the system 100. The data contained within such external database may be periodically updated. For example, new data may be added into the database (not shown in FIG. 1) and/or existing data may be modified and/or non-useful data may be deleted from the database. In one example, the data may be stored in an external system, such as a Lightweight Directory Access Protocol (LDAP) directory and a Relational Database Management System (RDBMS). Functions of the components of the system 100 are now explained with reference to the steps in flow diagrams in FIG. 2 and FIG. 3, and the example diagrams in FIG. 4A through 4G. Data used for the experiments were taken from Physionet 2017 challenge on single Short ECG lead recordings.

FIG. 2 is a flow diagram depicting steps involved in the process of generating 2D representation of ECG signals, using the system of FIG. 1, according to some embodiments of the present disclosure.

Steps in the method 200 are explained with reference to the components of the system 100 as depicted in FIG. 1. In an embodiment, the system 100 comprises one or more data storage devices or the memory 104 operatively coupled to the processor(s) 102 and is configured to store instructions for execution of steps of the method 200 by the processor(s) or one or more hardware processors 102. The steps of the method 200 of the present disclosure will now be explained with reference to the components or blocks of the system 100 as depicted in FIG. 1 and the steps of flow diagram as depicted in FIG. 2. Although process steps, method steps, techniques or the like may be described in a sequential order, such processes, methods, and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

In method 200, at step 202 of the method 200, a raw Electrocardiogram (ECG) signal is obtained as input, via one or more hardware processors 102. The system 100 may obtain the raw ECG directly from the user via appropriate sensors attached to the user. In an alternate embodiment, the system 100 may obtain the ECG signal from any other data source. Further, at step 204 of the method 200, the system 100 removes, via a bandpass filter implemented by the one or more hardware processors 102, noise data from the raw ECG signal to obtain a clean signal. Further, at step 206 of the method 200, the system 100 segments via the one or more hardware processors 102, the clean signal to obtain a plurality of segments, based on R-R intervals, wherein each of the plurality of segments comprises a left R-peak and a right R-peak. For example, consider the ECG signal in FIG. 4A, which includes a plurality of labelled R-peaks. As can be seen in FIGS. 4B and 4C, each segment has two consecutive R-peaks. The segment in FIG. 4B is obtained from the ECG signal in FIG. 4A and contains R-peaks 1 and 2, and is named as Segment1. The segment in FIG. 4C is obtained from the ECG signal in FIG. 4A and contains R-peaks 3 and 4, and is named as Segments.

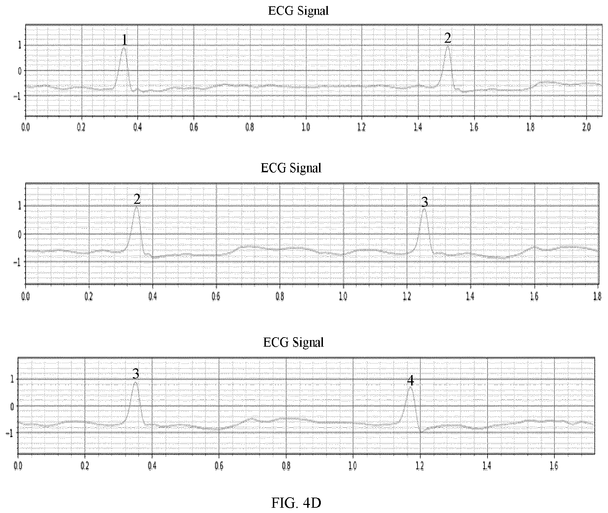

Further, at step 208 of the method 200, the system 100 arranges via the one or more hardware processors, the plurality of segments by aligning the left R-peak of the plurality of segments. This is depicted in FIG. 4D, which consists of individual Segments 1,2,3 respectively which are arranged one below the another. Interval between different R-peaks in the ECG signal maybe different, and this maybe an indication of a potential health condition of the user. The system 100, at step 210 of the method 200, determines via the one or more hardware processors 102, variability between the plurality of segments, in terms of position of the left R-peak and the right R-peak of consecutive segments. The term 'variability' in the context of the embodiments disclosed herein refers to difference in the interval between R-peaks for consecutive segments.

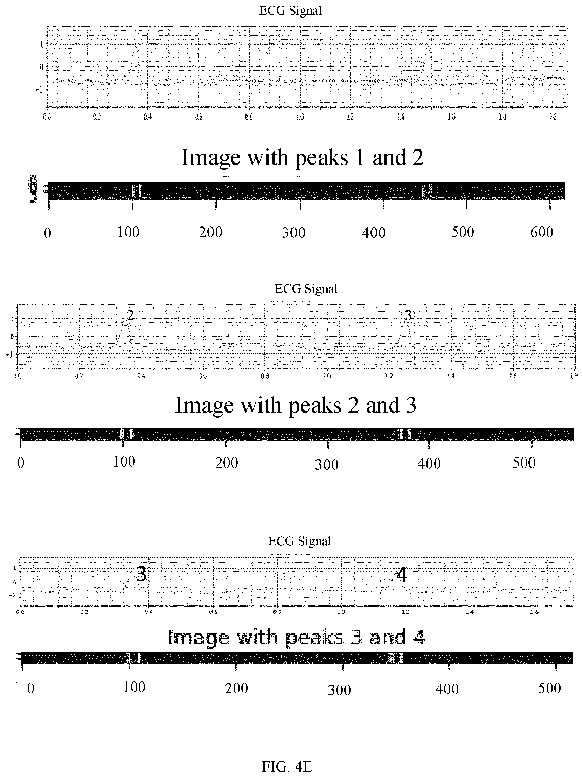

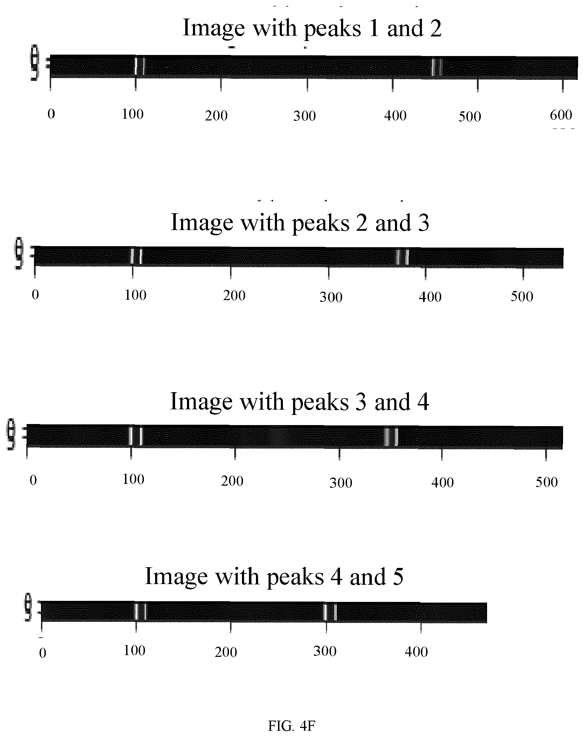

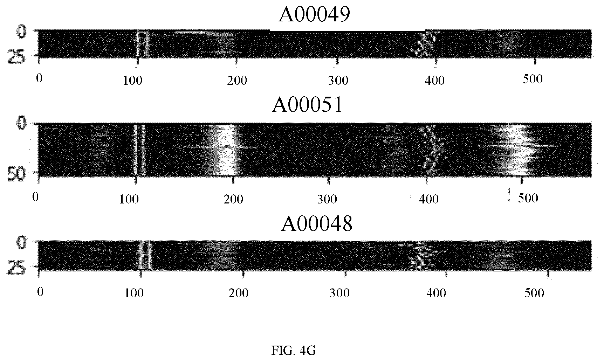

Further, at step 212 of the method 200, the system 100 generates, via the one or more hardware processors 102, 2-Dimensional (2D) representation of the segments, wherein in the 2D representation, information on relative position of the left R-peak and the right R-peak of each of the segments are captured. Generating the 2D representation includes transforming the plurality of segments that are 1-Dimensional format into a fixed 2D format, where each of a plurality of rows of the 2D format comprises of one left R-peak and one right R-peak and a corresponding morphological information, further wherein the 2D format comprises of a plurality of distinct and co-located R-peaks, and a plurality of P-waves representing the morphological information, where the plurality of R-peaks and the plurality of P-waves are separated in time axis based on the R-R intervals. The 2D representation establishes a temporal dependency between different R-peaks comprising the left R-peaks and right R-peaks of the plurality of segments, and represents relative positions between the R-peaks in different segments. FIG. 4E depicts the conversion of individual segment to corresponding image representation. FIG. 4F depicts the image representation for the segments 1,2,3,4 arranged one below the another. FIG. 4G depicts the representation of the original input ECG signal to the 2D image representation.

The system 100 is further configured to generate a data model using method 300 in FIG. 3. At step 302 of the method 300, the system 100 extracts a structural information from the 2D representation, by processing the 2D representation using an attention mechanism, wherein the structural information comprises information on a plurality of important regions of the ECG signal that contribute to classification of the ECG signal as being associated with one or more of a plurality of cardiovascular diseases (CVDs). For example, an area of the ECG signal where an abnormal spike is visible, maybe determined as an important region, by the system 100. Likewise other conditions maybe defined and configured with the system 100, so that the system 100 can interpret and identify one or more corresponding important regions. The system 100 then transforms the structural information into an ECG domain knowledge, wherein the ECG domain knowledge represents a determined classification of the ECG signal as being associated with one or more of the plurality of CVDs, i.e. the system 100 understands whether or not the ECG signal or a portion of the ECG signal represents one or more CVD conditions of the user, and if yes, which particular CVD(s) the user is associated with. In an embodiment, the ECG domain knowledge information maybe updated dynamically to include details of new/additional CVDs and/or to remove details of one or more CVDs that are already part of the domain knowledge, as required. Further, at step 306 of the method 300, the system 100 trains a data model by using a) the ECG signal, b) the extracted structural information, and c) information on the domain knowledge the structural information has been transformed to, as a training data.

The data model may then be used to process real-time ECG data inputs from one or more users, and determine whether the user is suffering from any of the CVDs, and accordingly generate output in a format that the user can interpret and understand. For example, the output maybe in verbal format i.e. a sequence of words.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of present disclosure herein addresses unresolved problem of interpretability of ECG signals by common users. The embodiment, thus provides a system that generates a 2-Dimensional (2D) representation of ECG signals. Moreover, the embodiments herein further provides a mechanism of generating a data model that can process real-time ECG signals and determine whether user is suffering from any Cardio Vascular Diseases (CVDs) and accordingly generate outputs in suitable format.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method (200), comprising:
obtaining (202), via one or more hardware processors, a raw Electrocardiogram (ECG) signal as input;
removing (204), via a bandpass filter implemented by the one or more hardware processors, noise data from the raw ECG signal to obtain a clean signal;
segmenting (206), via the one or more hardware processors, the clean signal to obtain a plurality of segments, wherein each of the plurality of segments comprises a left R-peak and a right R-peak;
arranging, via the one or more hardware processors, (208) the plurality of segments by aligning the left R-peak of the plurality of segments;
determining (210), via the one or more hardware processors, variability between the plurality of segments, in terms of position of the left R-peak and the right R-peak of consecutive segments; and
generating (212), via the one or more hardware processors, 2-Dimensional (2D) representation of the segments, wherein in the 2D representation comprises information on relative position of the left R-peak and the right R-peak of each of the segments are captured.

2. The method as claimed in claim 1, wherein generating the 2D representation comprises transforming the plurality of segments that are 1-Dinmnsional format are into a fixed dimension 2D format, where each of a plurality of rows of the 2D format comprises of one left R-peak and one right R-peak and a corresponding morphological information, further wherein the 2D format comprises of a plurality of distinct and co-located R-peaks, and a plurality of P-waves representing the morphological information, where the plurality of R-peaks and the plurality of P-waves are separated in time axis based on the R-R intervals.

3. The method as claimed in claim 1 further comprising:
extracting (302) a structural information from the 2D representation, by processing the 2D representation using an attention mechanism, wherein the structural information comprises information on a plurality of important regions of the ECG signal that contribute to classification of the ECG signal as being associated with one or more of a plurality of cardiovascular diseases (CVDs);
transforming (304) the structural information into an ECG domain knowledge, wherein the ECG domain knowledge represents a determined classification of the ECG signal as being associated with one or more of the plurality of CVDs; and
training (306) a data model by using a) the ECG signal, b) the extracted structural information, and c) information on the domain knowledge the structural information has been transformed to, as a training data.

4. The method as claimed in claim 1, wherein the clean signal is segmented based on R-R intervals.

5. The method as claimed in claim 1, wherein the 2D representation establishes a temporal dependency between different R-peaks comprising the left R-peaks and right R-peaks of the plurality of segments, and represents relative positions between the R-peaks in different segments.

6. A system (100), comprising:
one or more hardware processors (102);
a communication interface (112); and
a memory (104) comprising a plurality of instructions, wherein the plurality of instructions cause the one or more hardware processors to:
obtain a raw Electrocardiogram (ECG) signal as input;
remove via a bandpass filter implemented by the one or more hardware processors, noise data from the raw ECG signal to obtain a clean signal;
segment the clean signal to obtain a plurality of segments, wherein each of the plurality of segments comprises a left R-peak and a right R-peak;
arrange the plurality of segments by aligning the left R-peak of the plurality of segments;
determine variability between the plurality of segments, in terms of position of the left R-peak and the right R-peak of consecutive segments; and
generate 2-Dimensional (2D) representation of the segments, wherein in the 2D representation, information on relative position of the left R-peak and the right R-peak of each of the segments are captured.

7. The system as claimed in claim 6, wherein the one or more hardware processors are configured to generate the 2D representation by transforming the plurality of segments that are 1-Dinmnsional format into a fixed dimension 2D format, wherein in the fixed dimension 2D format, each of a plurality of rows comprises of one left R-peak and one right R-peak and a corresponding morphological information, further wherein the fixed 2D format comprises of a plurality of distinct and co-located R-peaks, and a plurality of P-waves representing the morphological information, where the plurality of R-peaks and the plurality of P-waves are separated in time axis based on the R-R intervals.

8. The system as claimed in claim 6, wherein the one or more hardware processors are configured to:
extract a structural information from the 2D representation, by processing the 2D representation using an attention mechanism, wherein the structural information comprises information on a plurality of important regions of the ECG signal that contribute to classification of the ECG signal as being associated with one or more of a plurality of cardiovascular diseases (CVDs);
transform the structural information into an ECG domain knowledge, wherein the ECG domain knowledge represents a determined classification of the ECG signal as being associated with one or more of the plurality of CVDs; and
train a data model by using a) the ECG signal, b) the extracted structural information, and c) information on the domain knowledge the structural information has been transformed to, as a training data.

9. The system as claimed in claim 6, wherein the one or more hardware processors are configured to segment the clean signal based on R-R intervals.

10. The system as claimed in claim 6, wherein the one or more hardware processors are configured to establish through the 2D representation, a temporal dependency between different R-peaks comprising the left peaks and right peaks of the plurality of segments, and wherein the 2D representation further represents relative positions between the R-peaks in different segments.

11. One or more non-transitory machine-readable information storage mediums comprising one or more instructions which when executed by one or more hardware processors cause:
obtaining a raw Electrocardiogram (ECG) signal as input;
removing via a bandpass filter implemented by the one or more hardware processors, noise data from the raw ECG signal to obtain a clean signal;
segmenting the clean signal to obtain a plurality of segments, wherein each of the plurality of segments comprises a left R-peak and a right R-peak;
arranging the plurality of segments by aligning the left R-peak of the plurality of segments;
determining variability between the plurality of segments, in terms of position of the left R-peak and the right R-peak of consecutive segments; and
generating 2-Dimensional (2D) representation of the segments, wherein in the 2D representation comprises information on relative position of the left R-peak and the right R-peak of each of the segments are captured.

12. The one or more non-transitory machine-readable information storage mediums as claimed in claim 11, wherein generating the 2D representation comprises transforming the plurality of segments that are 1-Dinmnsional format are into a fixed dimension 2D format, where each of a plurality of rows of the 2D format comprises of one left R-peak and one right R-peak and a corresponding morphological information, further wherein the 2D format comprises of a plurality of distinct and co-located R-peaks, and a plurality of P-waves representing the morphological information, where the plurality of R-peaks and the plurality of P-waves are separated in time axis based on the R-R intervals.

13. The one or more non-transitory machine-readable information storage mediums as claimed in claim 11 further comprising:
extracting a structural information from the 2D representation, by processing the 2D representation using an attention mechanism, wherein the structural information comprises information on a plurality of important regions of the ECG signal that contribute to classification of the ECG signal as being associated with one or more of a plurality of cardiovascular diseases (CVDs);
transforming the structural information into an ECG domain knowledge, wherein the ECG domain knowledge represents a determined classification of the ECG signal as being associated with one or more of the plurality of CVDs; and
training a data model by using a) the ECG signal, b) the extracted structural information, and c) information on the domain knowledge the structural information has been transformed to, as a training data.

14. The one or more non-transitory machine-readable information storage mediums as claimed in claim 11, wherein the clean signal is segmented based on R-R intervals.

15. The one or more non-transitory machine-readable information storage mediums as claimed in claim 11, wherein the 2D representation establishes a temporal dependency between different R-peaks comprising the left R-peaks and right R-peaks of the plurality of segments, and represents relative positions between the R-peaks in different segments.
